Europäisches Patentamt

European Patent Office

Office européen des brevets

(19)

(11) Veröffentlichungsnummer: **0 362 591**
**A1**

# EUROPÄISCHE PATENTANMELDUNG

(21) Anmeldenummer: **89117076.3**

(22) Anmeldetag: **15.09.89**

(51) Int. Cl.5 **A61B 5/107** , **G01B 11/24**

(30) Priorität: **17.09.88 DE 3831630**

(43) Veröffentlichungstag der Anmeldung:
**11.04.90 Patentblatt 90/15**

(84) Benannte Vertragsstaaten:
**AT BE CH DE ES FR GB GR IT LI NL SE**

(71) Anmelder: **Landwehr, Ulrich M.**
**Bahnhofstrasse 8**
**D-3000 Hannover 1(DE)**

(72) Erfinder: **Landwehr, Ulrich M.**
**Bahnhofstrasse 8**
**D-3000 Hannover 1(DE)**

(54) Verfahren und Vorrichtung zur Ermittlung der Abmessungen eines Gegenstandes auf photographischem Wege.

(57) Bei einem Verfahren zur Ermittlung der Abmessungen eines Gegenstandes auf photographischem Wege wird eine Photographie des Gegenstandes zusammen mit einem Meßraster erzeugt. Während der Aufnahme wird ein Muster aus horizontal verlaufenden Linien auf den Gegenstand projiziert. Das durch einen Projektor erzeugte Linienmuster wird durch einen Spiegel (4) derart reflektiert, daß alle Linien parallel zueinander und vorzugsweise mit gleichem Abstand zueinander reflektiert werden.

Fig 1

EP 0 362 591 A1

Die Erfindung betrifft ein Verfahren zur Ermittlung der Abmessungen eines Gegenstandes auf photographischem Wege, insbesondere der menschlichen Körpermaße, insbesondere der Oberflächenkontur eines Menschen, bei dem eine Photographie des Gegenstandes zusammen mit einem Meßraster hergestellt, und während der Aufnahme ein schräg von oben einfallendes Muster aus horizontal verlaufenden Linien mittels eines Projektors auf den Gegenstand projiziert wird und eine Vorrichtung zur Durchführung des Verfahrens.

Ein solches Verfahren ist zum Beispiel aus der DE-PS 29 48 101 sowie der DE-OS 34 25 913 bekannt. Das schräg von oben einfallende Linienmuster ermöglicht dabei die Ermittlung der dritten Dimension, d. h. Wölbungen und Senken in der Oberfläche des Körpers können sichtbar gemacht werden. Weichen z. B. die auf den Gegenstand projizierten Linien nach oben aus der Horizontalen ab, so befindet sich an dieser Stelle eine Erhebung. Ist das Linienmuster eng genug, läßt sich unter Zuhilfenahme des Meßrasters ein sehr genaues dreidimensionales Abbild des Gegenstandes ermitteln. Das Linienmuster wird bei den bekannten Verfahren durch einen Blitzlichtprojektor mittels eines Diapositivs erzeugt. Nachteilig ist jedoch, daß insbesondere bei der Vermessung von großen Körpern, z. B. eines Menschen, wo es erforderlich ist, zumindest den Rumpf darzustellen, die Linien unter verschiedenen Winkeln projiziert werden. Richtet man den Projektor so aus, daß seine optische Achse unter exakt 45° zur Vertikalen verläuft, sind die Projektionswinkel der oberhalb der optischen Achse verlaufenden Linien größer als 45°, die darunter verlaufenden kleiner als 45°. Um zu exakten Werten zu gelangen, müssen die auf den außerhalb der in der optischen Achse des Blitzlichtprojektors verlaufenden Linien gelegenen Meßpunkte mit einem Umrechnungsfaktor versehen werden. Dieser Nachteil führt dazu, daß eine Digitalisierung des Bildes mittels eines Computers nur schwer durchführbar ist.

Um diesen Nachteil aufzuheben, ist es aus der DE-OS 36 21 917 bekannt, eine Vielzahl von Blitzlichtprojektoren, die jeder ein eigenes Diapositiv aufweisen, übereinander anzuordnen. Die optischen Achsen aller Blitzlichtprojektoren verlaufen unter 45° zur Vertikalen. Die Abweichung der außerhalb der optischen Achse verlaufenden Linien ist hier recht gering, so daß schon eine einigermaßen exakte Darstellung der Oberfläche des Körpers möglich ist. Jedoch ist der Aufwand für eine solche Vorrichtung sehr hoch, und für viele Anwendungsfälle ist die geforderte Genauigkeit nicht gegeben.

Der Erfindung liegt von daher die Aufgabe zugrunde, das Verfahren der eingangs erwähnten Art dahingehend zu verbessern, daß ohne eine nennenswerte Erhöhung des Aufwandes eine wesentlich höhere Meßgenauigkeit erzielbar ist.

Diese Aufgabe wird dadurch gelöst, daß gemäß der Erfindung das Linienmuster mittels eines Spiegels reflektiert wird, dessen Oberfläche derart gekrümmt oder geneigt ist, daß die Linien des Linienmusters parallel zueinander vorzugsweise unter 45° und vorzugsweise mit gleichem Abstand zueinander auf den Gegenstand projiziert werden. Durch die Neigung bzw. Wölbung des Spiegels wird jede der unter einem anderen Winkel auf den Spiegel auftreffenden Linien exakt unter dem gleichen Winkel vorzugsweise von 45° auf den Körper reflektiert. Durch die Reflektion des Linienmusters ist mit extrem einfachen Mitteln die Lösung des in der Aufgabenstellung beschriebenen Problems gelöst.

Die Erfindung betrifft weiterhin eine Vorrichtung zur Durchführung des Verfahrens, die sich von der in der DE-PS 29 48 010 beschriebenen Vorrichtung dahingehend unterscheidet, daß die Objektive von Projektor und Kamera einander zugekehrt sind, und daß in den Strahlengang des Projektors ein Spiegel mit nach Art eines Hohlspiegels gekrümmter bzw. unterschiedlich geneigter Oberfläche angeordnet ist. Durch den Spiegel werden die divergierenden Linien derart reflektiert, daß sie parallel zueinander, vorzugsweise unter 45° auf den Gegenstand einfallen.

Mit besonderem Vorteil ist der Spiegel aus einer Vielzahl von einzelnen streifenartigen Elementen zusammengesetzt. Die Elemente sind um ihre Längsachse verstellbar. Jedes Element ist vorgesehen um eine oder mehrere Linien aus dem Linienbündel zu reflektieren. Die Elemente sind zweckmäßigerweise aus Spiegelglas gefertigt und sind an ihren Schmalseiten in einem Halterahmen um ihre Längsachse verstellbar angeordnet.

Eine andere vorteilhafte Ausgestaltung des Spiegels besteht darin, daß dieser die Form einer gewölbten Platte aufweist und unter ca. 22,5° zur Vertikalen angeordnet ist. Der Krümmungsradius der Platte bzw. Spiegels richtet sich nach der Brennweite des Projektorobjektivs. Der Winkel von 22,5° wird zwischen der Tangente, die an der Stelle des Spiegels verläuft, an der die in der optischen Achse des Projektors verlaufende Linie auf den Spiegel auftrifft und der Vertikalen gebildet.

Eine besonders vorteilhafte Ausgestaltung der erfindungsgemäßen Vorrichtung besteht darin, daß der Spiegel im Querschnitt gesehen an seiner, dem Projektor zugekehrten Oberfläche ein sägezahnartiges Profil aufweist, und daß die Steigung der Sägezähne derart variiert, daß jede der auf die Sägezahnflächen auftreffenden Linien parallel reflektiert wird.

Zweckmäßigerweise ist die Kamera eine Videokamera. Daraus ergibt sich der Vorteil, daß die erzeugten Bilder in einem an die Videokamera angeschlossenen Rechner gespeichert und jederzeit auf einem Bildschirm dargestellt werden können. Für diesen Anwendungsfall hat es sich als vorteilhaft erwiesen, anstelle des üblicherweise verwendeten Blitzlichtprojektors einen Dauerlichtprojektor zu verwenden.

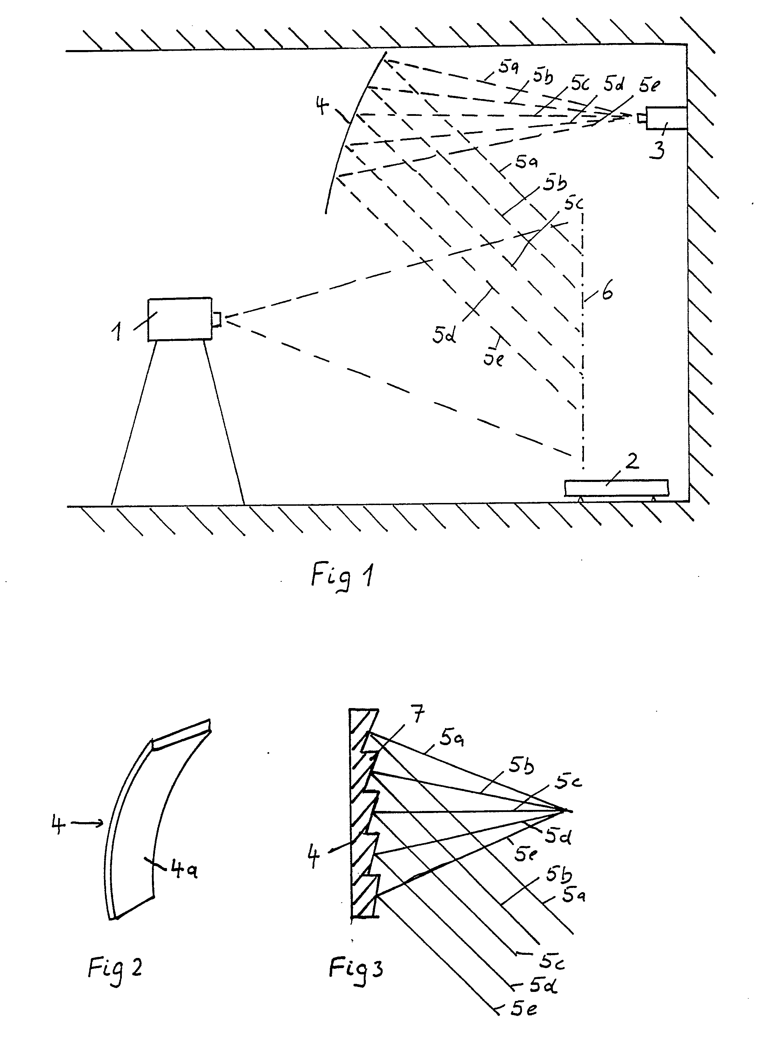

Die Erfindung ist an Hand der in den Figuren 1 bis 3 schematisch dargestellten Ausführungsbeispiels näher erläutert.

Die Figur 1 zeigt eine seitliche Ansicht einer erfindungsgemäßen Vorrichtung, die aus einer Kamera 1, z. B. einer Videokamera, die in nicht dargestellter Weise an einen Rechner angeschlossen ist, besteht. Die Kamera 1 dient dazu, eine Aufnahme eines auf einer Balanceplatte 2 postierten nicht dargestellten Menschen zu erzeugen, um dessen Oberfläche in drei Dimensionen vermessen zu können, wie es im Patent 29 48 010 beschrieben ist. Dazu wird gemeinsam mit dem Menschen ein nicht dargestelltes Meßraster auf der Aufnahme erzeugt. Das Meßraster wird vorteilhafterweise durch einen im Innern der Kamera befindlichen Projektor auf das Bildmaterial in der Kamera 1 projiziert. Eine solche Kamera ist in der DE-OS 36 32 450 beschrieben. Wird eine Videokamera verwendet, wird das Meßraster durch den Rechner erzeugt.

Zur Projektion der horizontal verlaufenden Höhenlinien wird ein Blitzlichtprojektor 3 verwendet, der das Linienmuster mittels eines nicht dargestellten Diapositivs erzeugt. Bei Verwendung einer Videokamera ist der Blitzlichtprojektor 3 zweckmäßigerweise ein Dauerlichtprojektor.

In dem Strahlengang des Blitzlichtprojektors 3 befindet sich ein Spiegel 4, dessen dem Projektor 3 zugekehrte Oberfläche derart gekrümmt ist, daß jede der auf den Spiegel 4 auftreffenden Linien 5a, 5b, 5c, 5d, und 5e unter dem gleichen Winkel reflektiert wird. Durch diese Parallelprojektion entfällt eine unterschiedliche Umrechnung der einzelnen Meßpunkte. Der Umrechnungsfaktor ist für die Meßpunkte der gleiche. Wird das Linienmuster unter 45° reflektiert, entfällt eine Umrechnung, d. h. die Auslenkung der Linie entspricht der Wölbung bzw. Senkung an dieser Stelle. Damit das Meßraster exakt auf die Meßebene 6, die als vertikal gedachte Ebene im vorderen Drittel der Balanceplatte 2 gebildet ist, ausgerichtet werden kann, befindet sich in dieser Ebene 6 am seitlichen Bildrand eine nicht dargestellte Meßlatte mit einer Skala.

Ein ebenfalls nicht dargestelltes Lot befindet sich in der Mitte der Bildebene ca. 50 cm vor der Meßebene 6. Dieses Lot dient dazu, daß Meßraster lotgerecht auszurichten und die Mittelsenkrechte auf Abweichungen zu kontrollieren.

Damit ein Linienmuster 5a bis 5e unter einem anderen Einfallswinkel als dargestellt erzeugt werden kann, ist vorgesehen, daß der Blitzlichtprojektor 3 und/oder der Spiegel 4 um die Horrizontale schwenkbar sind. Dies kann z. B. durch Fernsteuerung vorgenommen werden. Bei dem in der Figur 1 dargestellten geneigten Spiegel 4 ist ein korrigiertes Diapositiv erforderlich, welches für einen anzustrebenden gleichen Abstand der Höhenlinien sorgt.

Die Figur 2 zeigt einen Spiegel 4 in perspektivischer Darstellung, dessen Oberfläche 4a entsprechend gekrümmt ist, um die Parallelprojektion zu ermöglichen.

Die Figur 3 zeigt einen Schnitt durch eine besonders vorteilhafte Ausgestaltung eines Spiegels 4, der an seiner dem Blitzlichtprojektor 3 zugekehrten Oberfläche eine Vielzahl von sägezahnartigen Profilen 7 aufweist, deren Neigungswinkel zur Vertikalen von oben nach unten abnimmt und somit in besonders einfacher Weise eine Parallelprojektion ermöglicht. Bei dieser Ausführungsform kann auf ein korrigiertes Diapositiv verzichtet werden.

Zweckmäßigerweise ist für jede Linie 5a - 5e ein Sägezahnprofil 7 vorgesehen, jedoch besteht auch die Möglichkeit, weniger Sägezahnprofile 7 vorzusehen, so daß z. B. drei Linien von einem Sägezahnprofil reflektiert werden. Die Genauigkeit ist dann jedoch nicht so hoch. Diese Abweichung kann für manche Anwendungsfälle in Kauf genommen werden.

Die Spiegel 4 lassen sich in einfacher Weise herstellen, z. B. durch Ausgießen einer das Oberflächenprofil des Spiegels 4 aufweisenden Form. Durch Silberbedampfung läßt sich dann der Spiegel wirtschaftlich herstellen.

Wie an sich bekannt, lassen sich Kamera 1 und Balanceplatte 2 gemeinsam verfahren, so daß eine gute Anpaßbarkeit an eine unterschiedliche Körpergröße der zu vermessenden Personen gegeben ist. Eine besonders geeignete Balanceplatte ist in der DE-OS 37 14 015 beschrieben.

Für die Vermessung des Rumpfes eines erwachsenen Menschen reichen ca 90 cm Höhe des Linienmusters in Bereich der Meßebene 6 aus. Die Breite beträgt ca 60 cm und der Abstand der Linien zueinander ca 1 cm. Für Ausschnittaufnahmen kann durch Wechsel des Diapositivs auch ein geringerer Linienabstand verwendet werden. Bei der Kamera 1 wird dann ein Teleobjektiv eingesetzt.

## Ansprüche

1. Verfahren zur Ermittlung der Abmessungen eines Gegenstandes auf photographischem Wege, insbesondere der menschlichen Körpermaße, insbesondere der Oberflächenkontur eines Menschen, bei dem eine Photographie des Gegenstandes zusammen mit einem Meßraster hergestellt und während der Aufnahme ein schräg von oben einfallendes Muster aus horizontal verlaufenden Linien mittels eines Projektors auf den Gegenstand projiziert wird, dadurch gekennzeichnet, daß das Linienmuster mittels eines Spiegels reflektiert wird, dessen Oberfläche derart gekrümmt bzw. geneigt ist, daß die Linien des Linienmusters parallel zueinander, vorzugsweise unter 45° vorzugsweise mit gleichem Abstand zueinander auf den Gegenstand projiziert werden.

2. Vorrichtung zur Durchführung des Verfahrens nach Anspruch 1, bestehend aus einer Kamera und einem das Linienmuster mittels eines Diapositives erzeugenden Projektor, dadurch gekennzeichnet, daß die Objektive von Kamera (1) und Projektor (3) einander zugekehrt sind, und daß in dem Strahlengang des Projektors (3) ein Spiegel (4) mit nach Art eines Hohlspiegels gekrümmter bzw. unterschiedlich geneigter Oberfläche angeordnet ist.

3. Vorrichtung nach Anspruch 2, dadurch gekennzeichnet, daß der Spiegel (4) aus einer Vielzahl von einzelnen streifenartigen Elementen zusammengesetzt ist, daß die Elemente um ihre Längsachse verstellbar sind und daß jedes Element vorgesehen ist, eine oder mehrere Linien aus dem Linienbündel (5a - 5c) zu reflektieren.

4. Vorrichtung nach Anspruch 2, dadurch gekennzeichnet, daß der Spiegel (4) die Form einer gewölbten Platte aufweist, die unter ca. 22,5° zur Vertikalen geneigt angeordnet ist.

5. Vorrichtung nach Anspruch 2 oder einem der folgenden, dadurch gekennzeichnet, daß der Spiegel (4) im Querschnitt gesehen an seiner dem Projektor (3) zugekehrten Oberfläche ein sägezahnartiges Profil aufweist, und daß die Steigung der Sägezähne (7) derart variiert, daß jede der auf die Sägezahnflächen auftreffenden Linien (5a - 5c) parallel reflektiert wird.

6. Vorrichtung nach einem oder mehreren der Ansprüche 2 bis 5, dadurch gekennzeichnet, daß die Kamera (1) eine Videokamera ist.

EP 0 362 591 A1

Fig 1

Fig 2

Fig 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-A-3 638 941 (K. KREYENBERG) <br> * Spalte 2, Zeilen 8-20; Figur 1 * | 1 | A 61 B 5/107 <br> G 01 B 11/24 |
| A | --- | 6 | |
| Y | SYSTEMS & COMPUTERS IN JAPAN <br> Band 17, Nr. 12, Dezember 1986, Seiten 63-74, Silver Spring, Maryland, USA; J. OHYA: "Object Recognition Sensor Using Ring Pattern Method for Robotic Applications" * Figur 8 * | 1 | |
| A | idem <br> --- | 2,6 | |
| A | US-A-1 937 433 (G. MOE) <br> * Seite 2, Zeilen 75-111; Figuren 3-5 * <br> --- | 1,2 | |
| A | EP-A-0 253 185 (U. LANDWEHR) <br> * Zusammenfassung; Figur 1 * <br> ----- | 1 | |
| | | | **RECHERCHIERTE SACHGEBIETE (Int. Cl.5)** |
| | | | A 61 B 5/00 <br> G 01 B 11/00 <br> G 01 C 11/00 |

Der vorliegende Recherchenbericht wurde für alle Patentansprüche erstellt

| Recherchenort | Abschlußdatum der Recherche | Prüfer |
|---|---|---|
| BERLIN | 16-11-1989 | WEIHS J.A. |